# EUROPEAN PATENT APPLICATION

(11) **EP 0 839 529 A2**
(43) Date of publication of application: **06.05.1998**
(21) Application number: 97117896.7
(22) Date of filing: 13.03.1989
(51) Int. Cl.: A61K 31/215, A61K 31/16

(54) **Compositions containing aliphatic monocarboxylic acid esters or amides for the treatment of skin diseases**

(62) Divisional of application: 89905334.2
(71) Applicant: CELLEGY PHARMACEUTICALS, INC., Novato, CA 94949 (US)
(72) Inventor: Thornfeldt, Carl Richard, Ontario, Oregon 97914 (US)
(74) Representative: Sparing - Röhl - Henseler Patentanwälte

(57) **Abstract**

Skin conditions characterized by hyperactive mitochondria including inflammatory, hyperproliferative, hyperplastic, and dysplastic skin cells are treated with topical formulations of 5 to 19 carbon atom length aliphatic monocarboxylic acid esters or amides, as well as 5 to 19 carbon atom length monoaliphatic amines or their pharmaceutically acceptable salts.

## Description

This invention relates to a pharmaceutical composition for topical treatment of hyperactive, hyperplastic, hyperproliferative and dysplastic cells of the human skin. The invention resides in the discovery that esters and amides of monocarboxylic acids in which the acid moiety has 9 to 18 carbon atoms effectively treat acne, rosacea, psoriasis, seborrheic dermatitis, diaper dermatitis, numelar eczema, atopic eczema, contact eczema, warts, molluscum contagiosum, condylomatus carcinoma in situ, actinic (solar) keratoses. Bowen's disease, lentigo maligna, and melasma. The compounds according to the invention may function as primary or adjunctive therapeutic agents.

U.S. Patents Nos. 4,292,326 (Nazarro-Porro, September 29, 1981) and 4,386,104 (Nazarro-Porro, May 31, 1983) and 4,713,394 (Thornfeldt, Deember 15, 1987) disclose the use of certain dicarboxylic acids as therapeutic agents for a variety of skin diseases. U.S. Patent No. 4,067,997 (Kabara, January 10, 1978) discloses the activity against yeast, fungus, and bacteria of a synergistic combination of a 12 carbon atom monocarboxylic acid glycerol ester and a phenolic compound, used as a food preservative. U.S. A 4,406,884 (Fawzi) discloses C₅ and C₁₂ fatty acids and their use as topical compositions for the treatment of various skin diseases. US A 4,478,853 (Chaussee) discloses a composition for personal care use.

The documents makes no mention of esters and amides of monocarboxylic acids in which the acid moiety has 9 to 18 carbon atoms, having activity for the topical treatment of hyperactive, hyperplastic, hyperproliferative and dysplastic cells of the human skin.

Acne vulgaris is a multifactorial disease occurring in teenagers and young adults, with inflammatory and noninflammatory comedos on the face and upper trunk. The disease prerequisite is sebaceous glands activated by androgens. For some yet unknown reason hypercornification in the gland duct occurs blocking normal mobility of skin and follicle microorganisms. The restricted environment stimulates release of enzymes (lipases) by Propionobacterium Acnes (an anaerobic corynebacterium), Staphylococcus Epidermidis, and Pitrosporum Ovale (a yeast). Damage to the gland structure and surrounding tissue by the lipases results in inflammatory papules, pustules, and cysts. The comedos are free of these microbes. In some, the disease is only manifest as noninflammatory lesions but all patients with inflammatory lesions have some comedos. Major treatments consist of oral and topical antibiotics and retinoids; salicylic acid, sulfur, and benzoyl peroxide topically, and oral antiandrogen birth control pills.

Psoriasis is a multifactorial disease with epidermal hyperproliferation and epidermal and dermal inflammation producing the lesions. Microbes play an etiologic role since at least 50% of the patients carry Staphylococcus Aureus in the lesions. Beta hemolytic streptococcus is known to cause guttate psoriasis. The psoriasis lesions are sharply demarcated red with thick white scale. They occur predominately on knees, elbows, scalp, genitalia, and buttocks. Current treatments consist of topical corticosteroids, tar, anthralin, methotrexate, azathioprine, etretinate, psoralens plus ultraviolet A light, and tar plus ultraviolet B light.

Eczema is a descriptive term referring to poorly demarcated pruritic, erythematous, scaley, blistered, weeping, fissured or crusted lesions due to many causes. Atopic and numular are the most common types, afflicting any age group. Usually the lesions occur on the face, neck, and flexural surfaces. In most patients, there is heavy growth of Staphylococcus Aureus from the lesions of atopic and numular eczema. A purulent rapidly progressive variant, infectious eczematoid, is due to a mixed infection of Staphylococcus Aureus and Streptococcus Pyogenes or either bacteria alone. Current therapy includes topical and systemic corticosteroids, antipruritics, and antibiotics and topical tar.

Warts and molluscum contagiosum are hyperproliferative tumors due to epidermal cell invasion by the Human Papilloma virus and a pox virus, respectively. Unlike other skin virus infections that kill the invaded cells, both these viruses produce hyperplastic, hyperproliferative keratinocytes. Both viruses most commonly infect children. The wart tumors have different morphology depending upon the viral subtype and the thickness of the skin invaded. Molluscum contagiosum are always pearly papules with a central umbilication on an erythematous base. There are currently 23 different chemical and physical destructive treatments, most of which are painful, poorly effective, or may produce systemic toxicity. Poor treatment efficacy in both infections results primarily from the marked tissue hyperplasia induced by the virus. The H.P.V. virus is a proven cancer causing agent.

Seborrheic dermatitis is a histopathologically eczematous dermatosis characterized by poorly demarcated scaley erythematous patches with yellowish greasy scales. "Dandruff" is a mild form of this condition, localized to the scalp. This disease may involve any one, several, or all of the following sites: scalp, eyebrows, glabella, paranasal and chin folds, ears and retroauricular sulci, presternal interscapular regions, pubic regions, and intergluteal folds. Pityrosporum ovale, a yeast, has been shown to play a significant role in 75% of afflicted patients. Present therapy includes corticosteroids, tar, sulfur, and antibiotics, including antiyeast agents.

Actinic keratoses are superficial inflammatory tumors arising on sun exposed and irradiated skin. These tumors are the most common premalignant skin lesions. Each tumor is erythematous to brown with variable scaling. Current therapies include excisional and cryosurgery, and 5-fluorouracil cream. The treatments hurt and often produce cosmetically unacceptable pigmentary residua.

Bowen's disease is a superficial intraepidermal tumor of keratinocytes most commonly caused by ultraviolet irradiation. Approximately 5% metastasize. These tumors frequently cover large areas of the skin. They often develop from actinic keratosis. Current treatments consist of excisional and cryosurgery and 5-fluorouracil cream.

Lentigo maligna is a tumor of premalignant melanocytes in the epidermis, usually occurring on sun exposed facial skin of elderly patients. Up to 30% progress to invasive cancer. These tumors frequently cover large surface areas. Usually treatment is surgery, although it is claimed that azelaic acid is effective in some patients.

Melasma is a hyperipigmentation state of sunex-posed skin resulting from excessive hormonal stimulation of melanocytes producing pigment granule hyperplasia. This condition is usually activated by pregnancy or oral contraceptives, but may persist years after removal of this stimulus. The only current therapy is hydroquinone, which not only is poorly effective but may produce permanent depigmentation and rarely a paradoxical hyperpigmentation.

The conditions described above are the most common skin diseases and tumors, for which it has now been discovered that esters and amides of monocarboxylic acids in which the acid moiety has 9 to 18 carbon atoms effectively treat when applied topically.

The pharmaceutical composition according to the invention for topical treatment of hyperactive, hyperplastic, hyperproliferative and dysplastic cells of the human skin comprises 1 % to 35 % by weight with respect to the total composition of a compound selected from the group consisting of esters and amides of monocarboxylic acids in which the acid moiety has 9 to 18 carbon atoms, in association with a pharmaceutically acceptable vehicle that makes said composition suitable for topical application.

The compounds include straight-chain and branched-chain species, and saturated and unsaturated species, including species with multiple unsaturation sites. Examples for the monocarboxylic acid component of the esters and amides include pelargonic, capric, undecanoic, lauric, tridecanoic, myristic, myristoleic, palmitic, palmitoleic, hexadecanoic, oleic, linoleic, linolenic, and octadecanoic acids. The ester group includes glycerides and polyglycerides such as monoglycerides, triglycerides, hexaglycerides, and decaglycerides, and esters formed from methanol, ethanol, propylene glycol, polyethylene glycol, orethanol, and sorbitol, and saccharides such as sucrose. Specific examples include 1-monolaurin, 2-monolaurin, monocaprin, monomyristin, monolinolein; triglycerol caprylate, pelargonate, caprate, and laurate; hexaglycerol caproate, caprylate, pelargonate, caprate and laurate; decaglycerol butyrate, caprylate, pelargonate, caprate, and laurate; sucrose caprylate, caprate, laurate, myristate, palmitate, elaidate, oleate, and linoleate. Examples of amide are capratoyl-N,N-dimethylamide, lauryl-N,N-dimethylamide, myristoleyl-N,N-dimethylamide, and palmitoleyl-N,N-dimethylamide. A preferred example is lauryl-N,N-dimethylamide.

The compounds are generally applied in dermatological formulations. These include any of the various known mixtures and combinations which may be applied topically and will permit even spreading of the active ingredient over the affected area. Examples include creams, lotions, solutions, ointments, and unguents.

The concentration of the active ingredient in the formulation is not critical and may vary over a wide range. The concentration may indeed range as high as the upper limit of dissolvability in any given formulation. The concentration should be a therapeutically effective concentration, however, and in most cases, best results are achieved within a range of about 1% to about 35% by weight, preferably from about 2.5% to about 17.5% by weight.

The formulation may contain additional ingredients on an optional basis, including both those which are biologically active and those which are biologically inactive. Keratolytic agents are particularly useful in some cases as added active ingredients. Examples are salicylic acid, sulfur and retinoid derivatives. optional concentrations will vary among keratolytic agents. Salicylic acid, for example, is preferably used at about 0.5% to about 5.0% while sulfur is preferably used at about 2.0% to about 10.0%. Appropriate concentration ranges for any particular keratolytic agent will be apparent to those skilled in the art.

Stratum corneum penetration enhancing compounds are usually included in dermatologic formulations to boost efficacy. Examples include propylene glycol, sodium lauryl sulfate, dimethylamide, N-methyl-2-pyrrolidone, and Azone (Nelson Research, Irvine, California).

Examples of inactive ingredients are wetting agents, surfactants, emollients, and solvents.

The term "therapeutically effective amount" is used herein in terms of the amount of dermatological formulation to be applied in any particular case to denote any amount which will cause a substantial improvement in a disease condition (such as a subsidence of a lesion, for example) when applied to the affected area repeatedly over a period of time. The amount will vary with the condition being treated, the stage of advancement of the condition, and the type and concentration of formulaticn applied. Appropriate amounts in any given instance will be readily apparent to those skilled in the art or capable of determination by routine experimentation.

The compositions are generally applied in topical manner to the affected area, i.e., localized application to the skin region where the inflammation or hyperproliferation abnormality or tumor is manifest.

The following examples are offered for purposes of illustration, and are intended neither to define nor limit the invention in any manner.

Examples 1 through 4 illustrate the preparation of topical formulations in accordance with the present invention.

### EXAMPLE 1

A therapeutic ointment was prepared by dissolving 17.5 grams of 1-monolaurin (obtained from Lauricidin, Inc., Okemos, Michigan) in 10 mL of commercial isopropyl alcohol heated to 50°C. Commercial propylene glycol (7 mL) was the incorporated into the solution and the resulting mixture was cooled overnight at 24°C. The mixture was then worked into 100 g of Aquaphor (4-chloro-5-sulfamoyl-2',6'-salicyloxylidide, obtained from Beiersdorf, Inc., Norwalk Connecticut) on a pill tile.

The resulting ointment is hereinafter referred to as Formula A.

### EXAMPLE 2

A therapeutic ointment was prepared in a manner identical to that described in Example 1, except that 9 g of 1-monolaurin, 5 mL of isopropyl alcohol and 1 mL of propylene glycol were used.

The resulting ointment is hereinafter referred to as Formula B.

### EXAMPLE 3

A therapeutic formulation was prepared by dissolving 17.5 g of 1-monolaurin in 52 mL of isopropyl alcohol mixed with 24 mL of propylene glycol and heated to 50°C.

The resulting formulation is hereinafter referred to as Formula C.

### EXAMPLE 4

A therapeutic formulation was prepared in a manner identical to that described in Example 3, except that 24 g of 1-monolaurin, 52 mL of isopropyl alcohol and 24 mL of propylene glycol were used.

The resulting formulation is hereinafter referred to as Formula D.

Examples 5 through 10 illustrate the therapeutic effects of these formulations.

### EXAMPLE 5

Eleven patients with grade I-III acne vulgaris were treated twice daily with Formula C for 6 weeks. All these patients had failed to completely clear using all standard topical and therapeutic agents. Several had also failed using isotretinoin. With Formula C, however, seven of the patients cleared completely, two cleared more than 50% of their lesions, and two experienced mild worsening of their disease.

### EXAMPLE 6

Six patients with refractory plaque type psoriasis vulgaris were treated for four weeks twice daily with Formula A. These patients had failed to respond to all other topical and oral psoriasis treatments. As a result of the administration of Formula A, one patient completely cleared all skin lesions and the other five each experienced at least 75% clearing.

### EXAMPLE 7

Three patients with refractory facial seborrheic dermatitis were treated twice daily with Formula B for two weeks. These patients had previously failed to respond to topical corticosteroids, antifungals and antibiotics. As a result of the use of Formula B, all three gained complete resolution of the skin rash during the treatment period, and one of the three cleared in only three days.

### EXAMPLE 8

Three patients with refractory atopic dermatitis were treated with Formula A twice daily for six weeks. These patients had previously failed to respond to topical corticosteroids, tars, oral and topical antibiotics, and antihistamines. As a result of the use of Formula A, all three patients cleared completely during the treatment period.

### EXAMPLE 9

Two patients with refractory condyloma acuminata were treated with Formula D twice daily for six weeks. One completely cleared after two weeks of treatment; the other cleared after five weeks of treatment.

### EXAMPLE 10

Five patients with persistent melasma that had failed to be resolved by hydroquinone were treated for six weeks, twice daily, with Formula C. As a result, two of the five completely resolved, two others improved by 75%, and the remaining one by 50%.

The foregoing description is offered primarily for purposes of illustration. It will be readily apparent to those skilled in the art and numerous variations in both the formulations and their method of use, not mentioned above, may be made without departing from the spirit and scope of the invention.

## Claims

1. A pharmaceutical composition for topical treatment of hyperactive, hyperplastic, hyperproliferative and dysplastic cells of human skin, the composition comprising 1 % to 35 % by weight with respect to the total composition of a compound selected from the group consisting of esters and amides of monocarboxylic acids in which the acid moiety has 9 to 18 carbon atoms, in association with a pharmaceutically acceptable vehicle that makes said composition suitable for topical application.

2. The pharmaceutical composition in accordance to claim 1, wherein said pharmaceutical composition further compnses a stratum corneum penetration enhancer.

3. The pharmaceutical composition in accordance to claim 1 or 2, wherein said pharmaceutical composition further comprises a keratolytic agent.

4. The pharmaceutical composition in accordance to one of the claims 1 to 3. wherein said compound is selected from the group consisting of esters of pelargonic acid, capric acid undecanoic acid, lauric acid, tridecanoic acid myristic acid, myristoleic acid, palmitic acid, palmitoleic acid, hexadecanoic acid, oleic acid, linoleic acid and octadecanoic acid.

5. The pharmaceutical composition in accordance to one of the claims 1 to 4, wherein said compound is selected from the group consisting of esters of lauric acid.

6. The pharmaceutical composition in accordance to one of the claims 1 to 5, wherein said compound is an ester selected from the group consisting of glycerides, saccharides, esters of methanol, ethanol, propylene glycol, and polyethylene glycol.

7. The pharmaceutical composition in accordance to one of the claims 1 to 6, wherein said compound is an ester selected from the group consisting of monoglycerides, triglycerides, hexaglycerides, decaglycerides and sucrose esters.

8. The pharmaceutical composition in accordance to one of the claims 1 to 7, wherein said compound is selected from the group consisting of 1-monolaurin, 2-monolaurin, monocaprin, monomynstin. monolinolein, triglycerol caprylate, triglycerol pelargonate, triglycerol caprate, triglycerol laurate, hexaglycerol caproate, hexaglycerol caprylate, hexaglycerol pelargonate, hexaglycerol caprate, hexaglycerol laurate, decaglycerol butyrate, decaglycerol caprylate, decaglycerol pelargonate, decaglycerol caprate, decaglycerol laurate, sucrose myristate, sucrose palmitate, sucrose elaidate, sucrose oleate and sucrose linoleate.

9. The pharmaceutical composition in accordance to one of the claims 1 to 8, wherein said compound is 1-monolaurin.

10. The pharmaceutical composition in accordance to one of the claims 1 to 9, wherein said compound is an amide selected from the group consisting of capratoyl-N,N-dimethylamide, lauryl-N,N-dimethylamide, mynstoleyl-N,N-dimethylamide, palmitoleyl-N,N-dimethylamide, linoleyl-N,N-dimethylamide, and octadecanoyl-N,N-dimethylamide.

11. The pharmaceutical composition in accordance to one of the claims 1 to 10, wherein said compound is lauryl-N,N-dimethylamide.
